# EUROPEAN PATENT APPLICATION

(11) **EP 1 584 317 A1**
(43) Date of publication of application: **12.10.2005**
(21) Application number: 04447096.1
(22) Date of filing: 09.04.2004
(51) Int. Cl.: A61H 33/00, A61K 41/00

(54) **Apparatus for hydrotherapy or relaxation therapy**

(71) Applicant: Equilibrium N.V., 1851 Humsbeek (BE)
(72) Inventor: Van Assche, Yves, 1851 Humbeek (BE)
(74) Representative: Brants, Johan P.E.

(57) **Abstract**

The present invention relates to the use of a tachyonized product in an apparatus for hydrotherapy or relaxation therapy. Furthermore, the present invention relates to an apparatus for hydrotherapy or relaxation therapy comprising a tachyonized product.

## Description

### Field of the invention

This invention relates to an apparatus for hydrotherapy or relaxation therapy and to the treatment of the human or animal body for prevention and/or improving health conditions. The apparatus provides non-invasive, painless, relatively inexpensive yet effective modalities. In particular, the apparatus is provided with a "tachyonized product" which is incorporated in the matrix of the fabrication material of the apparatus or which is applied as outer coating onto said apparatus.

### Background of the invention

Healing was once a spiritual practice. Then modern medicine revolutionized the world with allopathic concepts and discoveries. A general evolution in awareness has witnessed people moving away from the chemical approach of allopathic medicine. Now over 60% of the world embraces alternative approaches to personal health.

These methods are based on one common principle: within our energetic continuum of subtle bodies, obstructions can manifest on any frequency level, and these obstructions then cause health problems. If one supplies the affected subtle frequency level with strengthening and harmonizing energy of its own frequency, these impediments can be dissolved and the state of health improved.

In the field of alternative healing therapies are known which are based on the creation of many different frequency oriented energy systems. For instance therapies based on photon energy, crystal energy, magnets, orgone energy, light acupuncture, radionic devices, photon lights, electromagnetic and sound therapy are known. Of course, all of these energy systems work well when they all have the potential ability to remove some blockages, to remove stagnant pockets where energy cannot flow freely in a certain layer of the subtle body system.

However, energetic frequency therapies, in general, have limits. One is that all these energies are limited to a certain frequency. Another limit is that the effect of any particular frequency is determined by the nature of that frequency. Photon energy, orgone energy, or electromagnetic energy, for example, can help because each has certain characteristics that forcefully affect the human body to move into another direction. However, this particular, specific frequency is not needed, such energies can also be harmful. It thus requires knowledge and experience to use these subtle energy devices safely and correctly.

Recently, the application of tachyon energy has been proposed. In itself, tachyon energy has stored all the potential needed to create a perfect energetic continuum in every individualized form of life. As this energy flows into a body's cells, it completely organizes the entire metabolism in a perfectly balanced manner. If any layer of the subtle body system is blocked, energy will not flow freely down into the next grosser level. From that level onward, there will be significant deficiencies of energy. Blockages in any of the subtle layers prevent tachyon energy from flowing freely into the material body.

Tachyon energy is totally different from the above mentioned energies. Tachyon energy is not a certain type of energy, but rather includes all energies in itself. Tachyon energy is not limited to a certain frequency and cannot be measured in the Hertzian frequency spectrum. In addition, tachyon energy cannot be influenced in any way whatsoever by any other form of energy. All other forms of subtle energies that are used in energetic medicine can be influenced, for example, by one's own thoughts and feelings.

The present invention aims to provide a novel type of application of tachyon energy for preventing and/or improving health conditions of a human or animal body.

More in particular, the present invention aims to provide an apparatus for use in hydrotherapy or relaxation therapy wherein tachyon energy is applied.

### Summary of the invention

In a first embodiment, the present invention relates to the use of a tachyonized product in an apparatus for hydrotherapy or relaxation therapy. In another embodiment, the present invention relates to an apparatus for hydrotherapy or relaxation therapy comprising a tachyonized product. The apparatus is preferably selected from the group comprising a bath, a shower, a shower cabinet, a hydrotherapy bath, a spa, a steam bath, a massaging device and the like.

Furthermore, the present invention also relates to a method for treating a living organism comprising exposing said organism in an apparatus for hydrotherapy or relaxation therapy to a tachyonized product, thereby preventing and/or improving health conditions of said organism.

The use of tachyonized products in an apparatus according to the present invention greatly improves health conditions and/or prevents the outcome of diseases. It results in a natural detoxification, increased absorption of available vitamins and minerals, increased energy for physical activities, increased awareness of subtle energies, increased brain function, increased circulation, exceptional improvements in athletic abilities and muscle recovery, etc. Furthermore, it can lead to the harmonization of physical, mental, emotional and spiritual levels in the human body.

In particular, the tachyonized product is applied in the apparatus according to the present invention by incorporating said product into the matrix of the fabrication material of said apparatus. Alternatively or in combination therewith, the tachyonized product may further be applied as a coating onto the outer surface of said apparatus.

In a preferred embodiment, the applied tachyonized product consists of a composition comprising a mixture of oxides. More preferably said tachyonized product consists of a composition comprising silicon dioxide, aluminium oxide, sodium oxide, potassium oxide, calcium oxide, iron oxide and magnesium oxide. In a particularly preferred embodiment, the tachyonized product consists of a composition comprising 60.62% w/v of silicon dioxide, 23.60% w/v of aluminium oxide, 10.50% of sodium oxide, 4.80 % w/v of potassium oxide 4.80%, 0.35M of calcium oxide, 0.08% w/v of iron oxide and 0.02% w/v of magnesium oxide.

### Detailed description of the invention

The term *"tachyon"* or *"tachyons"* as used herein refers to hypothetical faster-than-light particles, i.e. particles that travel at FTL (faster than light) speeds.

*"Tachyon energy'* as used herein refers to energy formed by sub-atomic particles having a faster than light speed.

The term *"tachyonization"* as used herein refers to the process of restructuring certain natural materials at the sub-molecular level. The molecular structure of the material changes thereby creating permanent tachyon antennas that are able to focus/attract tachyon energy. Once a material has been tachyonized, the rich flow of tachyon energy through the material will maintain the molecular alignment of the material permanently. The material will still look like the original host material, yet now is a permanent tachyon-energy antenna.

The term *"tachyonized product"* or *"tachyon-based product"* as used herein refers to a product that has been tachyonized such that it is able to work as an antenna that attracts tachyon energy.

In a preferred embodiment of the present invention, the applied tachyonized product consists of a composition comprising a mixture of oxides. More preferably said tachyonized product consists of a composition comprising silicon dioxide, aluminium oxide, sodium oxide, potassium oxide, calcium oxide, iron oxide and magnesium oxide. In a particularly preferred embodiment, the tachyonized product consists of a composition comprising 60.62% w/v of silicon dioxide, 23.60% w/v of aluminium oxide, 10.50% of sodium oxide, 4.80 % w/v of potassium oxide 4.80%, 0.35M of calcium oxide, 0.08% w/v of iron oxide and 0.02% w/v of magnesium oxide.

Preferably, said tachyonized product is applied in powder form. However, according to the invention, the tachyonized product may also be applied in the form of glass cells, silica disks, fabrics, fibre glass fabrics, liquids, etc. Non-limiting examples of tachyonized products include silica disk, a glass cell, a rubber flex100, a fibre glass mat, etc. In the latter case, the tachyonized product may be used as such or may be ground to form a powder and used in the form of a powder.

In an embodiment, the tachyonized product may be applied in the matrix for constructing the apparatus. In an example, the apparatus may be fabricated using fibre glass plates. In the latter case, the plates are either coated, dipped, painted, impregnated with the tachyonized product (powder). Alternatively, the tachyonized product may be used as a raw material in the fabrication of the plates and for instance the plates may be formed whereby a tachyonized product (e.g. a powder) is added during the fabrication process.

In a preferred embodiment, the tachyonized product is applied on the surface of the apparatus as an outer coating of said apparatus. Suitable techniques for applying the tachyonized product as a coating on the surface include but are not limited to painting, dipping, spraying or the like. Coating of an apparatus with a tachyonized product will generally induce a change in the appearance of the surface of the apparatus. More particularly, the tachyonized product will bleach the surface of the apparatus.

A tachyonized product can be identified by detecting its effects on a living organism. Therefore, the method according to Korotkov is used. This method, known as the Gas Discharge Visualization technique (GDV), is a breakthrough beyond Kirlian photography for direct, real-time viewing of the human aura and is well-known in the art. This technology allows one to capture by a special camera the physical, emotional, mental and spiritual energy emanating to and from an individual, plants, liquids, powders, inanimate objects and translate this into a computerized model. This allows researcher and client to see imbalances that may be influencing an individual's well-being greatly facilitating the diagnosis of the cause of any existing imbalances showing the area of the body and the organ systems involved. One of the greatest benefits to date is the ability to do "real-time" measurements of a variety of treatments for such conditions to determine which is the most appropriate.

Summarized, the technique is based on the principle of obtaining information, using the GDV (Computerised Gas Discharge Visualisation) technique. An instrument is applied based on well-known Kirlian Effect. This technique enables to visualize the distribution of human's fields, and make it easily, reproducibly, graphically and, the last but not the least, inexpensively. Electric impulse stimulates biological subject and generates response of the subject in the form of electron and photon emission. Due to a short electrical impulse used (10 mcs) the subject responses in a wide band of frequencies. Simultaneously, at the expense of superficial and volume heterogeneity of the object, space-time modulation of the applied electromagnetic field (EMF) takes place. Weak emission and photon radiation of the object increases owing to a gas discharge, generated in EMF. The glow of this discharge is transformed by optical and CCD system into a computer file. On the basis of the calculated parameters and diagnostic hypothesis a certain conclusion or diagnosis is made. The picture, formed after processing and transformations, reveals as a two-dimensional amplitude-modulated fractal image. To study this image methods of fractal, matrix and probability analysis, realised in the form of Windows based unique program complex, are used.

In a first aspect, the present invention relates to the use of a tachyonized product in an apparatus for hydrotherapy or relaxation therapy.

In a preferred embodiment, the present invention relates to the use of a tachyonized product, wherein said product is selected from the group comprising a bath, a shower, a shower cabinet, a hydrotherapy bath, a spa, a steam bath, a massaging device and the like.

Another preferred embodiment of the present invention relates to the use as described above, wherein said tachyonized product is used in powder form.

Another embodiment of the present invention relates to the use as described above, wherein said tachyonized product is incorporated in the matrix of the fabrication materials of the apparatus. Another embodiment of the present invention relates to the use as described above, wherein said tachyonized product is applied on the surface of the apparatus as an outer coating of said apparatus.

Another embodiment of the present invention relates to the use as described above, wherein said tachyonized product is applied as an outer coating onto said apparatus by painting, dipping, spraying or the like.

Another embodiment of the present invention relates to the use as described above, wherein said tachyonized product induces a change in the appearance of the surface of the apparatus.

Another embodiment of the present invention relates to an apparatus for use in hydrotherapy or relaxation therapy comprising a tachyonized product. The apparatus according to the present invention, comprising a tachyonized product, releases tachyon energy. Tachyon energy is faster than light and has the property to order disharmonies in the vibration frequencies of living organisms and to restore equilibrium in energy balances in a living organism. Preferably the apparatus is selected from the group comprising a bath, a shower, a shower cabinet, a hydrotherapy bath, a spa, a steam bath, a massaging device, etc ...

Another embodiment of the present invention relates to the apparatus as described above, wherein said tachyonized product is used in powder form.

Another embodiment of the present invention relates to an apparatus as described above, wherein said tachyonized product is incorporated in the matrix of the fabrication material of the apparatus.

Another embodiment of the present invention relates to an apparatus as described above, wherein said tachyonized product is applied on the surface of the apparatus as an outer coating of said apparatus.

Another embodiment of the present invention relates to an apparatus as described above, wherein said tachyonized product is applied as an outer coating onto said apparatus by painting, dipping, spraying or the like.

An example of an apparatus according to the present invention which comprises a tachyonized product is a spa. Such spa may comprise indoor or open-air spas, such as those spas designed to be positioned in close proximity with either indoor or outdoor family pools. The spas may include individual heaters and circulation systems for circulating hot water within. The spas may provide therapeutic aspects and advantages in their ability to massage and relax strained muscles by surrounding the body with circulating hot water and by providing hot water pressure jets to direct streams of hot water directly onto the strained muscles. Hot water spas also provide simple physical relaxation and leisure time enjoyment for those not necessarily desiring physical therapy or a hot water massage of tired or strained muscles, but simple seeking to relax in the privacy in their home or backyard.

In yet another embodiment, the present invention relates to a method for treating an living organism comprising exposing said organism in an apparatus for hydrotherapy or relaxation therapy to a tachyonized product, thereby preventing and/or improving health conditions of said organism. The body is composed of electrical, magnetic, and tachyon (subtle) energy. Tachyon energy also is referred to as Prana, Chi, Universal Life Force Energies, Aura, among other names. The body of a living organism is not only sensitive to, but is also energetically balanced by tachyon energy. Tachyon energy is primarily used to harmonize subatomic structures and use specific tools for therapeutic treatments. Tachyon energy strengthens the energetic field which is directly linked to the physical body. It then activates and intensifies the structure of the cells, reminding them of their perfect order.

The tachyonized therapy comprises four facts:
- the tachyonized energy is the source of all frequencies;
- the tachyonized energy is not a frequency itself;
- the tachyonized energy provides the energy fields directly with energy and
- the tachyonized energy contains the potential of the whole energetic continuum.
These are the four basic principles upon applying the tachyonized energy in a therapeutic way. They explain why the tachyonized products are curative and do not have side-effects. The energy of tachyonized products brings order into the system by means of degeneration.

It is not necessary to know at what point to stop the appliance of tachyonized energy: it reorganizes the energy fields and when the optimum structure of the energy fields is reached, the effect of the tachyonized energy stops by itself. Apart from frequency therapies, the use of tachyonized products has another advantage. A therapist who continuously works with tachyon energy will hardly ever take the energy from his or her clients. Due to the fact that tachyon does not have a specific frequency, it is not influenced by the gravity. This is an important aspect: no frequency matrix has the capacity of retaining or passing tachyon energy.

## Claims

1. Use of a tachyonized product in an apparatus for hydrotherapy or relaxation therapy.

2. Use of a tachyonized product according to claim 1, wherein said product is selected from the group comprising a bath, a shower, a shower cabinet, a hydrotherapy bath, a spa, a steam bath, a massaging device and the like.

3. Use according to claim 1 or 2, wherein said tachyonized product is used in powder form.

4. Use according to any of claims 1 to 3, wherein said tachyonized product is incorporated in the matrix of the fabrication material of the apparatus.

5. Use according to any of claims 1 to 4, wherein said tachyonized product is applied on the surface of the apparatus as an outer coating of said apparatus.

6. Use according to any of claims 1 to 5, wherein said tachyonized product is applied as an outer coating onto said apparatus by painting, dipping, spraying or the like.

7. Use according to any of claims 1 to 6, wherein said tachyonized product induces a change in the appearance of the surface of the apparatus.

8. Apparatus for hydrotherapy or relaxation therapy comprising a tachyonized product.

9. Apparatus according to claim 8, wherein said product is selected from the group comprising a bath, a shower, a shower cabinet, a hydrotherapy bath, a spa, a steam bath, a massaging device and the like.

10. Apparatus according to claim 8 or 9, wherein said tachyonized product is used in powder form.

11. Apparatus according to any of claims 8 to 10, wherein said tachyonized product is incorporated in the matrix of the fabrication material of the apparatus.

12. Apparatus according to any of claims 8 to 11, wherein said tachyonized product is applied on the surface of the apparatus as an outer coating of said apparatus.

13. Apparatus according to any of claims 8 to 12, wherein said tachyonized product is applied as an outer coating onto said apparatus by painting, dipping, spraying or the like.

14. A method for treating a living organism comprising exposing said organism in an apparatus for hydrotherapy or relaxation therapy to a tachyonized product, thereby preventing and/or improving health conditions of said organism.
